(19) Europäisches Patentamt
European Patent Office
Office européen des brevets

(11) Publication number: **0 130 527**
**B1**

# (12) EUROPEAN PATENT SPECIFICATION

(45) Date of publication of patent specification: **15.04.87**

(51) Int. Cl.$^4$: **C 07 F 9/10,** A 61 K 31/685

(21) Application number: **84107293.7**

(22) Date of filing: **26.06.84**

## (54) 1-0-Alkyl-3-aminopropan-1.2-diol-2-0-phospholipids, process for producing the same and pharmaceutical preparations containing the same.

(30) Priority: **02.07.83 DE 3323871**

(43) Date of publication of application:
**09.01.85 Bulletin 85/02**

(45) Publication of the grant of the patent:
**15.04.87 Bulletin 87/16**

(84) Designated Contracting States:
**AT BE CH DE FR GB IT LI LU NL SE**

(56) References cited:
**EP-A-0 092 190**
**GB-A-2 020 663**

(73) Proprietor: **A. Nattermann & Cie. GmbH**
**Nattermannallee 1**
**D-5000 Köln 30 (DE)**

(72) Inventor: **Lautenschläger, Hans-Heiner, Dr.**
**Neusser Gasse 50**
**D-5024 Pulheim-Stommeln (DE)**
Inventor: **Parnham, Michael J., Dr.**
**Aurikelweg 92**
**D-5024 Pulheim (DE)**
Inventor: **Prop, Gerrit, Dr.**
**Anemonenweg 23**
**D-5024 Pulheim-Sinnersdorf (DE)**

(74) Representative: **Redies, Bernd, Dr. rer. nat.**
**COHAUSZ & FLORACK Patentanwaltsbüro**
**Schumannstrasse 97 Postfach 14 01 47**
**D-4000 Düsseldorf 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention is related to 1-O-alkyl-3-amino-propane-1.2-diol-2-O-phospholipids, processes for producing the same and their use as active agents in drugs, in particular for the treatment of high blood pressure, atherosklerosis and asthma as well as for their application in connection with diseases of the immuno system and for the treatment of cancer.

The 1-O-1-alkyl-3-aminopropane-1.2-diol-2-O-phospholipids according to the present invention correspond to the formula I

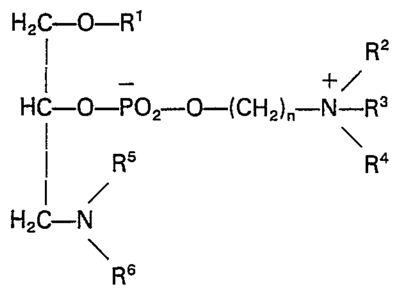

I

wherein

$R^1$ is a saturated or unsaturated, straight or branched chain acyclic hydrocarbyl group with 10 to 20 carbon atoms,

$R^2$, $R^3$ and $R^4$ which may be the same or different, represent hydrogen or an alkyl group with 1 to 4 carbon atoms,

$R^5$ and $R^6$ which may be the same or different, represent hydrogen or the group $—A—C_mH_{2m}—R^7$ or $—A—C_mH_{2m-2}—R^7$,

$R^7$ is hydrogen, unsubstituted phenyl or phenyl substituted by a $C_{1-3}$-alkyl, $C_{1-3}$-alkoxy or trifluoromethyl group, or a halogen atom,

A is a bond, —CO—, —COO— or $—CONR^8—$,

$R^8$ is hydrogen or $C_{1-4}$-alkyl,

m is a numeral from 0 to 20 and

n is a numeral from 2 to 4.

Preferred are those compounds of formula I wherein $R^1$ is a straight-chain alkyl group with 10 to 20 carbon atoms or a straight-chain alkenyl group with 1 or 2 double bonds and 10 to 20 carbon atoms, $R^2$, $R^3$ and $R^4$ which may be the same or different from each other, represent hydrogen or a methyl group, $R^5$ and $R^6$ which may be the same or different from each other, represent hydrogen or the group $—A—(CH_2)_m—R^7$, $R^7$ is hydrogen, unsubstituted phenyl or phenyl substituted by a methyl, methoxy or trifluoromethyl group or a halogen atom, A represents a bond, —CO—, —COO— or $—CONR^8—$, $R^8$ is hydrogen or $C_{1-4}$-alkyl, m is an integer from 0 to 20, if $R^7$ is hydrogen, or is an integer from 0 to 2, if $R^7$ is phenyl unsubstituted or substituted as indicated, and n is 2. Most preferred under those compounds are the compounds of formula I wherein $R^1$ is a straight-chain alkyl group having from 10 to 20 carbon atoms while $R^2$, $R^3$, $R^4$, $R^5$, $R^6$, $R^7$, $R^8$, m and n have the same meaning as indicated in connection with the preferred group of compounds.

Compounds according to the present invention are for instance:

3-Amino-1-O-decylpropane-1.2-diol-2-O-phosphocholine
3-Amino-1-O-undecylpropane-1.2-diol-2-O-phosphocholine
3-Amino-1-O-dodecylpropane-1.2-diol-2-O-phosphocholine
3-Amino-1-O-tridecylpropane-1.2-diol-2-O-phosphocholine
3-Amino-1-O-tetradecylpropane-1.2-diol-2-O-phosphocholine
3-Amino-1-O-pentadecylpropane-1.2-diol-2-O-phosphocholine
3-Amino-1-O-hexadecylpropane-1.2-diol-2-O-phosphocholine
3-Amino-1-O-heptadecylpropane-1.2-diol-2-O-phosphocholine
3-Amino-1-O-octadecylpropane-1.2-diol-2-O-phosphocholine
3-Amino-1-O-nonadecylpropane-1.2-diol-2-O-phosphocholine
3-Amino-1-O-eicosylpropane-1.2-diol-2-O-phosphocholine
1-O-Decyl-3-methylaminopropane-1.2-diol-2-O-phosphocholine
1-O-Dodecyl-3-methylaminopropane-1.2-diol-2-O-phosphocholine
3-Methylamino-1-O-tetradecylpropane-1.2-diol-2-O-phosphocholine
1-O-Hexadecyl-3-methylaminopropane-1.2-diol-2-O-phosphocholine
3-Methylamino-1-O-octadecylpropane-1.2-diol-2-O-phosphocholine
1-O-Eicosyl-3-methylaminopropane-1.2-diol-2-O-phosphocholine
1-O-Decyl-3-ethylaminopropane-1.2-diol-2-O-phosphocholine
1-O-Dodecyl-3-ethylaminopropane-1.2-diol-2-O-phosphocholine
3-Ethylamino-1-O-tetradecylpropane-1.2-diol-2-O-phosphocholine
3-Ethylamino-1-O-hexadecylpropane-1.2-diol-2-O-phosphocholine

3-Ethylamino-1-O-octadecylpropane-1.2-diol-2-O-phosphocholine
1-O-Eicosyl-3-ethylaminopropane-1.2-diol-2-O-phosphocholine
1-O-Decyl-3-hexadecylaminopropane-1.2-diol-2-O-phosphocholine
1-O-Dodecyl-3-hexadecylaminopropane-1.2-diol-2-O-phosphocholine
3-Hexadecylamino-1-O-tetradecylpropane-1.2-diol-2-O-phosphocholine
1-O-Hexadecyl-3-hexadecylaminopropane-1.2-diol-2-O-phosphocholine
3-Hexadecylamino-1-O-octadecylpropane-1.2-diol-2-O-phosphocholine
1-O-Eicosyl-3-hexadecylaminopropane-1.2-diol-2-O-phosphocholine
1-O-Decyl-3-octadecylaminopropane-1.2-diol-2-O-phosphocholine
1-O-Dodecyl-3-octadecylaminopropane-1.2-diol-2-O-phosphocholine
3-Octadecylamino-1-O-tetradecylpropane-1.2-diol-2-O-phosphocholine
1-O-Octadecyl-3-octadecylaminopropane-1.2-diol-2-O-phosphocholine
1-O-Eicosyl-3-octadecylaminopropane-1.2-diol-2-O-phosphocholine
1-O-Decyl-3-eicosylaminopropane-1.2-diol-2-O-phosphocholine
1-O-Dodecyl-3-eicosylaminopropane-1.2-diol-2-O-phosphocholine
3-Eicosylamino-1-O-tetradecylpropane-1.2-diol-2-O-phosphocholine
3-Eicosylamino-1-O-octadecylpropane-1.2-diol-2-O-phosphocholine
1-O-Eicosyl-3-eicosylaminopropane-1.2-diol-2-O-phosphocholine
3-Benzylamino-1-O-decylpropane-1.2-diol-2-O-phosphocholine
3-Benzylamino-1-O-dodecylpropane-1.2-diol-2-O-phosphocholine
3-Benzylamino-1-O-tetradecylpropane-1.2-diol-2-O-phosphocholine
3-Benzylamino-1-O-hexadecylpropane-1.2-diol-2-O-phosphocholine
3-Benzylamino-1-O-octadecylpropane-1.2-diol-2-O-phosphocholine
3-Benzylamino-1-O-eicosylpropane-1.2-diol-2-O-phosphocholine
1-O-Hexadecyl-3-phenylaminopropane-1.2-diol-2-O-phosphocholine
1-O-Octadecyl-3-phenylaminopropane-1.2-diol-2-O-phosphocholine
3-Acetylamino-1-O-decylpropane-1.2-diol-2-O-phosphocholine
3-Acetylamino-1-O-dodecylpropane-1.2-diol-2-O-phosphocholine
3-Acetylamino-1-O-tetradecylpropane-1.2-diol-2-O-phosphocholine
3-Acetylamino-1-O-hexadecylpropane-1.2-diol-2-O-phosphocholine
3-Acetylamino-1-O-octadecylpropane-1.2-diol-2-O-phosphocholine
3-Acetylamino-1-O-eicosylpropane-1.2-diol-2-O-phosphocholine
3-Butyrylamino-1-O-hexadecylpropane-1.2-diol-2-O-phosphocholine
3-Butyrylamino-1-O-octadecylpropane-1.2-diol-2-O-phosphocholine
3-Butyrylamino-1-O-eicosylpropane-1.2-diol-2-O-phosphocholine
1-O-Hexadecyl-3-palmitoylaminopropane-1.2-diol-2-O-phosphocholine
1-O-Octadecyl-3-palmitoylaminopropane-1.2-diol-2-O-phosphocholine
1-O-Eicosyl-3-palmitoylaminopropane-1.2-diol-2-O-phosphocholine
1-O-Hexadecyl-3-stearoylaminopropane-1.2-diol-2-O-phosphocholine
1-O-Octadecyl-3-stearoylaminopropane-1.2-diol-2-O-phosphocholine
1-O-Eicosyl-3-stearoylaminopropane-1.2-diol-2-O-phosphocholine
1-O-Hexadecyl-3-methoxycarbonylaminopropane-1.2-diol-2-O-phosphocholine
3-Methoxycarbonylamino-1-O-octadecylpropane-1.2-diol-2-O-phosphocholine
1-O-Eicosyl-3-methoxycarbonylaminopropane-1.2-diol-2-O-phosphocholine
3-Ethoxycarbonylamino-1-O-hexadecylpropane-1.2-diol-2-O-phosphocholine
3-Ethoxycarbonylamino-1-O-octadecylpropane-1.2-diol-2-O-phosphocholine
1-O-Eicosyl-3-ethoxycarbonylaminopropane-1.2-diol-2-O-phosphocholine
3-Benzyloxycarbonylamino-1-O-hexadecylpropane-1.2-diol-2-O-phosphocholine
3-Benzyloxycarbonylamino-1-O-octadecylpropane-1.2-diol-2-O-phosphocholine
3-Benzyloxycarbonylamino-1-O-eicosylpropane-1.2-diol-2-O-phosphocholine
1-O-Hexadecyl-3-ureidopropane-1.2-diol-2-O-phosphocholine
1-O-Octadecyl-3-ureidopropane-1.2-diol-2-O-phosphocholine
1-O-Eicosyl-3-ureidopropane-1.2-diol-2-O-phosphocholine
1-O-Hexadecyl-3-(3-methylureido)-propane-1.2-diol-2-O-phosphocholine
3-(3-Methylureido)-1-O-octadecylpropane-1.2-diol-2-O-phosphocholine
1-O-Eicosyl-3-(3-methylureido)-propane-1.2-diol-2-O-phosphocholine
3-(3-Ethylureido)-1-O-hexadecylpropane-1.2-diol-2-O-phosphocholine
3-(3-Ethylureido)-1-O-octadecylpropane-1.2-diol-2-O-phosphocholine
1-O-Eicosyl-3-(3-ethylureido)-propane-1.2-diol-2-O-phosphocholine
1-O-Hexadecyl-3-(3-hexadecylureido)-propane-1.2-diol-2-O-phosphocholine
3-(3-Hexadecylureido)-1-O-octadecylpropane-1.2-diol-2-O-phosphocholine
1-O-Eicosyl-3-(3-hexadecylureido)-propane-1.2-diol-2-O-phosphocholine
3-(3,3-Dimethylureido)-1-O-hexadecylpropane-1.2-diol-2-O-phosphocholine
3-(3,3-Dimethylureido)-1-O-octadecylpropane-1.2-diol-2-O-phosphocholine
3-(3,3-Dimethylureido)-1-O-eicosylpropane-1.2-diol-2-O-phosphocholine

3-(3-Benzylureido)-1-O-hexadecylpropane-1.2-diol-2-O-phosphocholine
3-(3-Benzylureido)-1-O-octadecylpropane-1.2-diol-2-O-phosphocholine
3-(3-Benzylureido)-1-O-eicosylpropane-1.2-diol-2-O-phosphocholine
3-(N-Acetylmethylamino)-1-O-hexadecylpropane-1.2-diol-2-O-phosphocholine
3-(N-Acetylmethylamino)-1-O-octadecylpropane-1.2-diol-2-O-phosphocholine
3-(N-Acetylmethylamino)-1-O-eicosylpropane-1.2-diol-2-O-phosphocholine
3-(N-Acetylmethylamino)-1-O-oleylpropane-1.2-diol-2-O-phosphocholine
3-(N-Acetylmethylamino)-1-O-linolylpropane-1.2-diol-2-O-phosphocholine
3-(N-Benzoylmethylamino)-1-O-hexadecylpropane-1.2-diol-2-O-phosphocholine
3-(N-Benzoylmethylamino)-1-O-octadecylpropane-1.2-diol-2-O-phosphocholine
3-(N-Benzoylmethylamino)-1-O-eicosylpropane-1.2-diol-2-O-phosphocholine
3-(N-Benzoylmethylamino)-1-O-oleylpropane-1.2-diol-2-O-phosphocholine
3-(N-Benzoylmethylamino)-1-O-linolylpropane-1.2-diol-2-O-phosphocholine
3-[N-(4-Chlorobenzoyl)-methylamino]-1-O-hexadecylpropane-1.2-diol-2-O-phosphocholine
1-O-Hexadecyl-3-[N-(4-methoxybenzoyl)-methylamino]-propane-1.2-diol-2-O-phosphocholine
1-O-Hexadecyl-3-[N-(4-methylbenzoyl)-methylamino]-propane-1.2-diol-2-O-phosphocholine
3-[N-(4-Ethoxybenzoyl)-methylamino]-1-O-hexadecyl-propane-1.2-diol-2-O-phosphocholine
1-O-Hexadecyl-3-[N-(3-trifluormethylbenzoyl)-methylamino]-propane-1.2-diol-2-O-phosphocholine
3-(N-Acetylhexadecylamino)-1-O-hexadecylpropane-1.2-diol-2-O-phosphocholine
3-(N-Acetylhexadecylamino)-1-O-octadecylpropane-1.2-diol-2-O-phosphocholine
3-(N-Acetylhexadecylamino)-1-O-eicosylpropane-1.2-diol-2-O-phosphocholine
3-(N-Acetylbenzylamino)-1-O-hexadecylpropane-1.2-diol-2-O-phosphocholine
3-(N-Acetylbenzylamino)-1-O-octadecylpropane-1.2-diol-2-O-phosphocholine
3-(N-Acetylbenzylamino)-1-O-eicosylpropane-1.2-diol-2-O-phosphocholine
1-O-Hexadecyl-3-(N-methylpalmitoylamino)-propane-1.2-diol-2-O-phosphocholine
3-(N-Methylpalmitoylamino)-1-O-octadecylpropane-1.2-diol-2-O-phosphocholine
1-O-Eicosyl-3-(N-methylpalmitoylamino)-propane-1.2-diol-2-O-phosphocholine
3-(N-Methylpalmitoylamino)-1-O-oleylpropane-1.2-diol-2-O-phosphocholine
3-(N-Methyloleoylamino)-1-O-oleylpropane-1.2-diol-2-O-phosphocholine
1-O-Hexadecyl-3-(N-octadecyloleoylamino)-propane-1.2-diol-2-O-phosphocholine
3-(N-Ethoxycarbonylmethylamino)-1-O-hexadecylpropane-1.2-diol-2-O-phosphocholine
3-(N-Ethoxycarbonylmethylamino)-1-O-octadecylpropane-1.2-diol-2-O-phosphocholine
1-O-Eicosyl-3-(N-ethoxycarbonylmethylamino)-propane-1.2-diol-2-O-phosphocholine
3-(N-Benzyloxycarbonylmethylamino)-1-O-hexadecylpropane-1.2-diol-2-O-phosphocholine
3-(N-Benzyloxycarbonylmethylamino)-1-O-octadecylpropane-1.2-diol-2-O-phosphocholine
3-(N-Benzyloxycarbonylmethylamino)-1-O-eicosylpropane-1.2-diol-2-O-phosphocholine
3-(N-Benzyloxycarbonylhexadecylamino)-1-O-hexadecylpropane-1.2-diol-2-O-phosphocholine
3-(N-Benzyloxycarbonylhexadecylamino)-1-O-octadecylpropane-1.2-diol-2-O-phosphocholine
3-(N-Benzyloxycarbonylhexadecylamino)-1-O-eicosylpropane-1.2-diol-2-O-phosphocholine
3-(N-Benzyloxycarbonyloctadecylamino)-1-O-hexadecylpropane-1.2-diol-2-O-phosphocholine
3-(N-Benzyloxycarbonyloctadecylamino)-1-O-octadecylpropane-1.2-diol-2-O-phosphocholine
3-(N-Benzyloxycarbonyloctadecylamino)-1-O-eicosylpropane-1.2-diol-2-O-phosphocholine
3-(N-Benzyloxycarbonyleicosylamino)-1-O-hexadecylpropane-1.2-diol-2-O-phosphocholine
3-(N-Benzyloxycarbonyleicosylamino)-1-O-octadecylpropane-1.2-diol-2-O-phosphocholine
3-(N-Benzyloxycarbonyleicosylamino)-1-O-eicosylpropane-1.2-diol-2-O-phosphocholine
3-(N-Benzylbenzyloxycarbonylamino)-1-O-hexadecylpropane-1.2-diol-2-O-phosphocholine
3-(N-Benzylbenzyloxycarbonylamino)-1-O-octadecylpropane-1.2-diol-2-O-phosphocholine
3-(N-Benzylbenzyloxycarbonylamino)-1-O-eicosylpropane-1.2-diol-2-O-phosphocholine
3-(N-Benzylbenzyloxycarbonylamino)-1-O-oleylpropane-1.2-diol-2-O-phosphocholine
1-O-Hexadecyl-3-(1-methylureido)-propane-1.2-diol-2-O-phosphocholine
3-(1-Methylureido)-1-O-octadecylpropane-1.2-diol-2-O-phosphocholine
1-O-Eicosyl-3-(1-methylureido)-propane-1.2-diol-2-O-phosphocholine
3-(1-Ethylureido)-1-O-hexadecylpropane-1.2-diol-2-O-phosphocholine
3-(1-Ethylureido)-1-O-octadecyl-propane-1.2-diol-2-O-phosphocholine
1-O-Eicosyl-3-(1-ethylureido)-propane-1.2-diol-2-O-phosphocholine
1-O-Hexadecyl-3-(1-hexadecylureido)-propane-1.2-diol-2-O-phosphocholine
3-(1-Hexadecylureido)-1-O-octadecylpropane-1.2-diol-2-O-phosphocholine
1-O-Eicosyl-3-(1-hexadecylureido)-propane-1.2-diol-2-O-phosphocholine
3-(1,3-Dimethylureido)-1-O-hexadecylpropane-1.2-diol-2-O-phosphocholine
3-(1,3-Dimethylureido)-1-O-octadecylpropane-1.2-diol-2-O-phosphocholine
3-(1,3-Dimethylureido)-1-O-eicosylpropane-1.2-diol-2-O-phosphocholine
3-(1-Benzylureido)-1-O-hexadecylpropane-1.2-diol-2-O-phosphocholine
3-(1-Benzylureido)-1-O-octadecylpropane-1.2-diol-2-O-phosphocholine
3-(1-Benzylureido)-1-O-eicosylpropane-1.2-diol-2-O-phosphocholine
1-O-Hexadecyl-3-(1-hexadecyl-3-methylureido)-propane-1.2-diol-2-O-phosphocholine

3-(1-Hexadecyl-3-methylureido)-1-O-octadecylpropane-1.2-diol-2-O-phosphocholine
1-O-Eicosyl-3-(1-hexadecyl-3-methylureido)-propane-1.2-diol-2-O-phosphocholine
1-O-Hexadecyl-3-(3-methyl-1-octadecylureido)-propane-1.2-diol-2-O-phosphocholine
3-(3-Methyl-1-octadecylureido)-1-O-octadecylpropane-1.2-diol-2-O-phosphocholine
1-O-Eicosyl-3-(3-methyl-1-octadecylureido)-propane-1.2-diol-2-O-phosphocholine
3-(3-Ethyl-1-hexadecylureido)-1-O-hexadecylpropane-1.2-diol-2-O-phosphocholine
3-(3-Ethyl-1-hexadecylureido)-1-O-octadecylpropane-1.2-diol-2-O-phosphocholine
1-O-Eicosyl-3-(3-ethyl-1-hexadecylureido)-propane-1.2-diol-2-O-phosphocholine
1-O-Hexadecyl-3-(1-hexadecyl-3-phenylureido)-propane-1.2-diol-2-O-phosphocholine
3-[3-(4-chlorophenyl)-1-hexadecylureido]-1-O-hexadecylpropane-1.2-diol-2-O-phosphocholine
3-(1-Hexadecyl-3-phenylureido)-1-O-octadecylpropane-1.2-diol-2-O-phosphocholine
1-O-Eicosyl-3-(1-hexadecyl-3-phenylureido)-propane-1.2-diol-2-O-phosphocholine
1-O-Hexadecyl-3-(3-phenylureido)-propane-1.2-diol-2-O-phosphocholine
1-O-Octadecyl-3-(3-phenylureido)-propane-1.2-diol-2-O-phosphocholine
1-O-Eicosyl-3-(3-phenylureido)-propane-1.2-diol-2-O-phosphocholine
3-[3-(4-chlorophenyl)-ureido]-1-O-hexadecylpropane-1.2-diol-2-O-phosphocholine
3-[3-(4-chlorophenyl)-ureido]-1-O-octadecylpropane-1.2-diol-2-O-phosphocholine
3-[3-(4-chlorophenyl)-ureido]-1-O-eicosylpropane-1.2-diol-2-O-phosphocholine
1-O-Hexadecyl-3-[3-(2-phenylethyl)-ureido]-propane-1.2-diol-2-O-phosphocholine
1-O-Octadecyl-3-[3-(2-phenylethyl)-ureido]-propane-1.2-diol-2-O-phosphocholine
1-O-Eicosyl-3-[3-(2-phenylethyl)-ureido]-propane-1.2-diol-2-O-phosphocholine
1-O-Hexadecyl-3-(2-phenylethylamino)-propane-1.2-diol-2-O-phosphocholine
1-O-Octadecyl-3-(2-phenylethylamino)-propane-1.2-diol-2-O-phosphocholine
1-O-Eicosyl-3-(2-phenylethylamino)-propane-1.2-diol-2-O-phosphocholine
3-(N-Acetyl-2-phenylethylamino)-1-O-hexadecylpropane-1.2-diol-2-O-phosphocholine
3-(N-Acetyl-2-phenylethylamino)-1-O-octadecylpropane-1.2-diol-2-O-phosphocholine
3-(N-Acetyl-2-phenylethylamino)-1-O-eicosylpropane-1.2-diol-2-O-phosphocholine
3-(N-Acetyl-2-phenylethylamino)-1-O-oleylpropane-1.2-diol-2-O-phosphocholine
[1-(N-Acetylaminomethyl)-2-hexadecyloxyethyl]-3-trimethylammoniopropylphosphate
[1-(N-Acetylaminomethyl)-2-hexadecyloxyethyl]-4-trimethylammoniobutylphosphate
[1-(N-Acetylmethylaminomethyl)-2-hexadecyloxyethyl]-3-trimethylammoniopropylphosphate
[1-(N-Acetylmethylaminomethyl)-2-hexadecyloxyethyl]-4-trimethylammoniobutylphosphate
[1-(N-Acetylaminomethyl)-2-octadecyloxyethyl]-3-trimethylammoniopropylphosphate
[1-(N-Acetylaminomethyl)-2-octadecyloxyethyl]-4-trimethylammoniobutylphosphate
[1-(N-Acetylmethylaminomethyl)-2-octadecyloxyethyl]-3-trimethylammoniopropylphosphate
[1-(N-Acetylmethylaminomethyl)-2-octadecyloxyethyl]-4-trimethylammoniobutylphosphate
[1-(N-Acetylaminomethyl)-2-hexadecyloxyethyl]-2-dimethylammonioethylphosphate
[1-(N-Acetyl-methylaminomethyl)-2-hexadecyloxyethyl]-2-dimethylammonioethylphosphate
[1-(N-Acetyl-aminomethyl)-2-octadecyloxyethyl]-2-dimethylammonioethylphosphate
[1-(N-Acetylmethylaminomethyl)-2-octadecyloxyethyl]-2-dimethylammonioethylphosphate
[1-(N-Acetylaminomethyl)-2-hexadecyloxyethyl]-2-butyldimethylammonioethylphosphate
[1-(N-Acetylaminomethyl)-2-octadecyloxyethyl]-2-butyldimethylammonioethylphosphate.

Depending upon whether the racemates have been separated or not, the above compounds may be present in their R- or S-form or as racemate mixtures.

The compounds of the present invention are biologically very active and may be used for instance in drugs or in plant protection. Thus, they may be used for the treatment of high blood pressure and for the therapy of cancer.

For preparing the new 1-O-alkyl-3-aminopropane-1.2-diol-2-O-phospholipids, 2.3-epoxypropyl ethers of formula

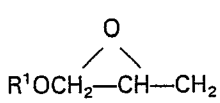

(regarding their production see E. Mouzin et al., Synthesis *1983,* 117 and following) are reacted with the corresponding amines of formula

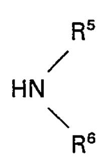

corresponding to the procedure known for epoxides (see for instance Houben-Weyl, Methoden der organischen Chemie, 4. Ed., Vol. 11/1, p. 314 and following, Georg Thieme Verlag, Stuttgart 1957) to yield

the corresponding substituted 3-amino-2-hydroxypropyl ethers and, if desired, subjecting them to N-acylation by usual methods. The starting materials II

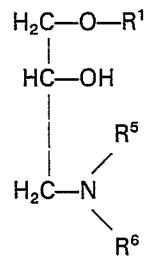

II

wherein $R^1$, $R^5$ and $R^6$ have the same meaning as in formula I, resulting therefrom are reacted with dichlorophosphoric acid ω-haloalkyl esters of formula III

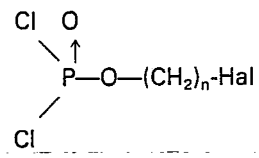

III

wherein n has the same meaning as in formula I and Hal is a chlorine or bromine atom, in an inert organic solvent, possibly with the addition of an auxiliary base such as pyridine or triethylamine, the resulting compounds subsequently being reacted with an amine of formula IV

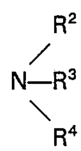

IV

wherein $R^2$, $R^3$ and $R^4$ have the same meaning as in formula I in an inert organic solvent such as toluene, dioxane, tetrahydrofuran, possibly with the application of pressure (regarding thereto, see: H. K. Mangold, Angew. Chemie *91*, 550 to 560 (1979; H. Eibl, Chem. and Phys. of Lipids *26*, 405 to 429 (1980)).

If the resulting compounds of formula I have benzyl, benzyloxycarbonyl or similar protective groups, these groups may be split-off by hydrogenation under usual conditions in the presence of heavy metal catalysts and hydrogen, thus forming compounds of formula I wherein $R^5$ and/or $R^6$ is hydrogen.

On the other hand, a compound of formula I wherein $R^5$ and/or $R^6$ is hydrogen may be reacted, in the presence of a suitable condensation agent such as thionyl chloride, carbonylbisimidazole and carbodiimides, with an acid of formula V

$$HOOC{-}(CH)_m{-}R^7 \qquad V$$

wherein m and $R^7$ have the same meaning as in formula I, or, possibly in the presence of auxiliary basis such as triethylamine and pyridine, with an acid derivative of formula VI

$$Hal\text{-}A{-}(CH_2)_m{-}R^7 \qquad VI$$

wherein A, m and $R^7$ have the same meaning as in formula I (with the exception of A representing a bond) and Hal is a halogen atom or an acid anhydride group, in particular a chloro or a bromo atom. Acylation may also be effected with isocyanates of formula VII

$$R^9{-}N{=}C{=}O \qquad VII$$

wherein $R^9$ is $R^7C_mH_{2m}$— or $R^7$ $C_mH_{2m-2}$—, wherein $R^7$ and m have the same meaning as in formula I, optionally with the addition of Lewis base catalysts such as dimethylformamide or 4-dimethylaminopyridine.

The starting compounds of formula II may be used in their R- or S-form or as racemates; accordingly, there are obtained the R- or S-forms or racemate mixtures of the final products I.

The present invention is further related to pharmaceutical preparations which contain the 1-O-alkyl-3-amino-propane-1.2-diol-2-O-phospholipids of formula I. The pharmaceutical preparations according to the present invention are products for enteral as oral or rectal as well as parenteral application. They contain the pharmaceutically active compounds alone or together with usual, pharmaceutically applicable carrier materials. Preferably, the pharmaceutical preparations are in the form of single doses corresponding to the

desired form of application such as tablets, dragees, capsules, suppositories, granulates, solutions, emulsions or suspensions. The dosages of the compounds usually are between 1 and 1000 mg. per day, preferably between 10 and 100 mg. per day, and the product may be administered once or several times, preferably between two and three times, per day.

The preparation of the compounds according to the present invention are further illustrated by the following Examples. The reported melting points have been determined by means of a Büchi 510 melting point apparatus and they are not corrected. The infrared spektra have been determined using a Perkin-Elmer 257 or Nicolet NIC-3600 type apparatus.

## Example 1

3-(N-acetylmethylamino)-1-O-hexadecylpropane-1.2-diol-2-O-phosphocholine.

a) 1-Hexadecyloxy-3-methylaminopropan-2-ol.

A cooled mixture of 15.5 g of methylamine, 50 ml of tetrahydrofuran and 15 g of hexadecyl 2.3-epoxypropyl ether is heated to 60°C for 2 hours in an autoclave, cooled and evaporated in vacuo. The residue is purified by column chromatography (silicic acid gel//chloroform/methanol).

Yield: 10.5 g mpt.: 89 to 91°C.

b) 3-(N-acetylmethylamino)-1-hexadecyloxypropan-2-ol.

5 g of 1-hexadecyloxy-3-methylaminopropan-2-ol are dissolved in 30 ml of anhydrous chloroform. At first 3 g of triethylamine and separately thereafter 2.4 g of acetyl chloride are added dropwise with cooling and the mixture is stirred for 8 hours. The chloroform solution is washed with 2% hydrochloric acid and water, is evaporated and the residue is dissolved in 100 ml of methanol. A solution of 0.6 g of sodium hydroxide in a little methanol is added to the methanol solution and the mixture is stirred at room temperature for one hour. The solvent is evaporated in vacuo and the residue is triturated with chloroform. The chloroform solution is washed with 2% hydrochloric acid and water is dried over sodium sulfate and is evaporated.

Yield: 3.7 g of an oil.
IR (film): 3350, 1630 1120 $cm^{-1}$.

c) [1-(N-acetylmethylaminomethyl)-2-hexadecyloxyethyl]-2-bromoethyl phosphate.

3.3 g of 3-(N-acetylmethylamino)-1-hexadecyloxypropan-2-ol are dissolved in 100 ml of anhydrous chloroform and the solution is added dropwise to a mixture of 4.3 g of 2-bromoethylphosphoric acid dichloride, 10 ml of chloroform and 50 ml of pyridine cooled with ice. The resulting mixture is stirred for one hour at room temperature, diluted with water and stirred another hour at room temperature. The organic phase is separated, washed with 5% hydrochloric acid and water, dried over sodium sulfate and the solvent is separated in vacuo. The residue is purified by column chromatography (silicic acid gel// chloroform/methanol).

Yield: 1.6 g of an oil.

d) 3-(N-acetylmethylamino)-1-O-hexadecylpropane-1.2-diol-2-O-phosphocholine.

1.6 g of [1-(N-acetylmethylaminomethyl)-2-hexadecyloxyethyl]-2-bromoethyl phosphate are dissolved in 30 cc of anhydrous toluene. About 3 ml of a 33% solution of trimethylamine in ethanol is added thereto and the resulting mixture is stirred for 4 hours at 60°C in a closed container. The solvent is evaporated in vacuo and the residue is purified by column chromatography (silicic acid gel//chloroform/methanol).

Yield: 0.4 g of a waxy product.
IR (film): 1635 $cm^{-1}$.

## Example 2

3-(N-benzylbenzyloxycarbonylamino)-1-O-hexadecyl-propane-1.2-diol-2-O-phosphocholine.

a) 3-Benzylamino-1-hexadecyloxypropan-2-ol.

A mixture of 24.4 g of benzylamine, 100 ml of tetrahydrofuran and 34 g of hexadecyl 2.3-epoxypropyl ether is refluxed for 8 hours and the solvent is evaporated in vacuo. The residue is recrystallized from hexane.

Yield: 30.8 g. Mpt: 56 to 58°C.

b) 3-(N-benzylbenzyloxycarbonylamino)-1-hexadecyloxypropan-2-ol.

21.4 g of 3-benzylamino-1-hexadecyloxypropan-2-ol are dissolved in 100 ml of anhydrous chloroform. At first 5.4 g of triethylamine and then a solution of 9 g of benzyl chloroformate in 50 ml of chloroform is added dropwise with cooling and the mixture is stirred for 3 hours. The chloroform solution is washed with

5% hydrochloric acid and water, dried over sodium sulfate, evaporated and the residue is purified by column chromatography (silicic acid gel//chloroform).

Yield: 21 g (oil).
IR (film): 3345, 1701, 1125 $cm^{-1}$.

c) [1-(N-benzylbenzyloxycarbonylaminomethyl)-2-hexadecyloxyethyl]-2-bromoethyl phosphate.
16 g of 3-(N-benzylbenzyloxycarbonylamino)-1-hexadecyloxypropan-2-ol are dissolved in 30 ml of anhydrous chloroform and the solution is added dropwise to an ice-cooled mixture of 14.5 g of 2-bromoethylphosphoric acid dichloride, 120 ml of chloroform and 9.5 g of pyridine. The mixture is stirred for 1 hour at room temperature, diluted with water and again stirred for 1 hour at room temperature. The organic phase is separated, washed with 5% hydrochloric acid and water, dried over sodium sulfate and the solvent is evaporated in vacuo. The residue is purified by column chromatography (silicic acid gel// chloroform/methanol).

Yield: 11.8 g (oil).

d) 3-(N-benzylbenzyloxycarbonylamino)-1-O-hexadecylpropane-1.2-diol-2-O-phosphocholine.
11.5 g of [1-(N-benzylbenzyloxycarbonylaminomethyl)-2-hexadecyloxyethyl]-2-bromoethyl phosphate are dissolved in 50 ml of anhydrous toluene. About 10 ml of a 33% solution of trimethylamine in ethanol is added thereto and the mixture is stirred for 4 hours at 60°C in a closed container. The solvent is evaporated in vacuo and the residue is purified by column chromatography (silicic acid gel//chloroform/methanol).

Yield: 4.6 g of a waxy product.
IR (film): 1696 $cm^{-1}$.

Example 3

3-Amino-1-O-hexadecylpropane-1.2-diol-2-O-phosphocholine.

4.3 g of 3-(N-benzylbenzyloxycarbonylamino)-1-O-hexadecylpropane-1.2-diol-2-O-phosphocholine are dissolved in 200 ml of a 4:1 (v/v)-mixture of dioxane and water and, after the addition of 0.43 g of palladium-active carbon, are hydrogenated with hydrogen. The solution is filtered, the filter residue is washed with ethanol, the filtrates are combined and evaporated to dryness. The residue is purified by column chromatography (silicic acid gel//chloroform/methanol/conc. ammonia).

Yield: 1.4 g. Mpt.: 217 to 219°C.

Example 4

3-Benzylamino-1-O-hexadecylpropane-1.2-diol-2-O-phosphocholine as produced similarly to Example 3 by incomplete hydrogenation.

For instance, there are isolated with the procedure described in Example 3 0.3 g of waxy 3-benzylamino-1-O-hexadecylpropane-1.2-diol-2-O-phosphocholine as side product. With the same procedure there of course may be also produced the 3-alkylamino-, 3-arylamino- and 3-arylalkylamino-compounds.

Example 5

3-Acetylamino-1-O-hexadecylpropane-1.2-diol-2-O-phosphocholine.

a) 0.35 g of 3-amino-1-O-hexadecyl-propane-1.2-diol-2-O-phosphocholine are dissolved in 10 ml of anhydrous chloroform. 0.14 g of acetic acid anhydride are added thereto and the mixture is stirred for about 12 hours at room temperature. The solution is evaporated in vacuo and the residue is purified by column chromatography (silicic acid gel//chloroform/methanol/water).

Yield: 0.2 g. Mpt.: 237 to 239°C.
IR (in KBr): 1667 $cm^{-1}$.

b) 0.24 g of 3-amino-1-O-hexadecylpropane-1.2-diol-2-O-phosphocholine are dissolved in 5 ml of anhydrous chloroform. 0.1 g of triethylamine and 0.08 g of acetyl chloride are added thereto and the mixture is stirred for about 12 hours at room temperature. The solution is evaporated in vacuo and the residue is purified by column chromatography (silicic acid gel//chloroform/methanol/water).

Yield: 0.18 g.

Example 6

1-O-Hexadecyl-3-(3-methylureido)-propane-1.2-diol-2-O-phosphocholine.

0.48 g of 3-amino-1-O-hexadecylpropane-1.2-diol-2-O-phosphocholine are dissolved in 10 ml of

anhydrous chloroform. 0.17 g of methyl isocyanate are added to this solution and the mixture is stirred for 12 hours at room temperature. The solution is evaporated in vacuo and the residue is purified by column chromatography (Silicic acid gel//chloroform/methanol/water).

Yield: 0.4 g. Mpt.: 243 to 245°C.

**Claims for the Contracting States: BE CH DE FR GB IT LI LU NL SE**

1. 1-O-Alkyl-3-aminopropane-1.2-diol-2-O-phospholipids of the formula I

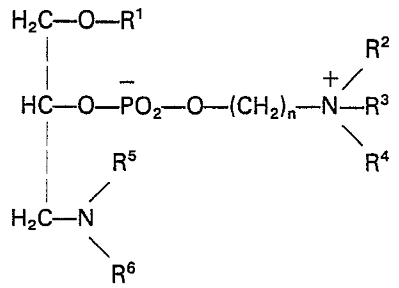

I

wherein

$R^1$ is a saturated or unsaturated, straight or branched chain acyclic hydrocarbyl group with 10 to 20 carbon atoms,

$R^2$, $R^3$ and $R^4$ which may be the same or different, represent hydrogen or an alkyl group with 1 to 4 carbon atoms,

$R^5$ and $R^6$ which may be the same or different, represent hydrogen or the group $—A—C_mH_{2m}—R^7$ or $—A—C_mH_{2m-2}—R^7$,

$R^7$ is hydrogen, unsubstituted phenyl or phenyl substituted by a $C_{1-3}$-alkyl, $C_{1-3}$-alkoxy or trifluoromethyl group, or a halogen atom,

A is a bond, —CO—, —COO— or $—CONR^8—$,

$R^8$ is hydrogen or $C_{1-4}$-alkyl,

m is a numeral from 0 to 20 and

n is a numeral from 2 to 4.

2. 1-O-Alkyl-3-aminopropane-1.2-diol-2-O-phospholipids of the general formula I according to claim 1 wherein

$R^1$ is a saturated or unsaturated, straight-chain acyclic hydrocarbyl group with 10 to 20 carbon atoms,

$R^2$, $R^3$ and $R^4$ which may be the same or different, represent hydrogen or methyl,

$R^5$ and $R^6$ which may be the same or different, represent hydrogen or the group $—A—(CH_2)_m—R^7$,

$R^7$ is hydrogen, unsubstituted phenyl or phenyl substituted by a methyl, methoxy, or trifluoromethyl group, or a halogen atom,

A is a bond, —CO—, —COO— or $—CONR^8—$,

$R^8$ is hydrogen or $C_{1-4}$-alkyl,

m is a numeral from 0 to 20, if $R^7$ is hydrogen, or is a numeral from 0 to 2, if $R^7$ is phenyl or substituted phenyl and

n is 2.

3. Process for producing the compounds of formula I as claimed in claims 1 and 2 characterized in that a 2.3-epoxypropylether of formula

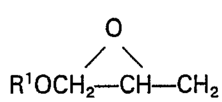

wherein $R^1$ is as defined in claims 1 and 2, is reacted with an amine of formula

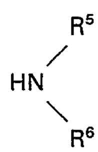

wherein $R^5$ and $R^6$ are as defined in claims 1 and 2, after optional N-acylation, reacting the resulting compound of the formula II

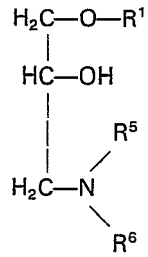

II

wherein $R^1$, $R^5$ and $R^6$ are as defined in claims 1 and 2, with a dichlorophosphoric acid ω-haloalkyl ester of formula III

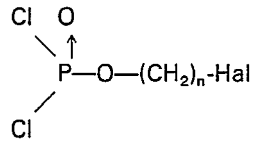

III

wherein n is as defined in claims 1 and 2 and Hal is a chlorine or bromine atom, in an inert organic solvent, optionally in the presence of an auxiliary base, and reacting the resulting product with an amine of formula IV

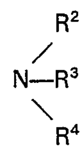

IV

wherein $R^2$, $R^3$ and $R^4$ are as defined in claims 1 and 2, in an inert organic solvent, optionally under pressure.

4. Process for the preparation of compounds of formula I as claimed in claims 1 or 2 wherein $R^5$ and/or $R^6$ are hydrogen characterized in that a compound of formula I wherein one or both of $R^5$ and/or $R^6$ represent a benzyl or benzyloxycarbonyl group, is hydrogenated in an inert solvent in the presence of a hydrogenation catalyst.

5. Process for the production of compounds of formula I as claimed in claims 1 or 2 characterized in that a compound of formula I wherein $R^5$ and/or $R^6$ are hydrogen, is reacted with an acid of formula V

$$HOOC—(CH_2)_m—R^7 \qquad V$$

wherein m and $R^7$ are as defined in claims 1 and 2 in the presence of suitable condensation agents or, optionally in the presence of auxiliary bases, with an acid derivative of formula VI

$$Hal\text{-}A—(CH_2)_m—R^7 \qquad VI$$

wherein A, m and $R^7$ are as defined in claims 1 and 2, except A does not represent a bond, and Hal is a halogen atom or an acid anhydride group, or, optionally in the presence of a Lewis base as catalyst, is reacted with an isocyanate of formula VII

$$R^9—N=C=O \qquad VII$$

wherein $R^9$ is $R^7C_mH_{2m}$— or $R^7C_mH_{2m-2}$—, wherein $R_7$ and m are as defined in claim 1.

6. Pharmaceutical preparations characterized in that they contain a compound of general formula I as claimed in claims 1 and 2 as the active ingredient mixed with usual pharmaceutical auxiliaries and carriers.

**Claim for the Contracting State: AT**

Process for producing the compounds of the formula I

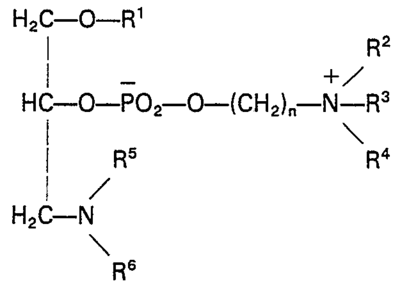

I

wherein

$R^1$ is a saturated or unsaturated, straight or branched chain acyclic hydrocarbyl group with 10 to 20 carbon atoms,

$R^2$, $R^3$ and $R^4$ which may be the same or different, represent hydrogen or an alkyl group with 1 to 4 carbon atoms,

$R^5$ and $R^6$ which may be the same or different, represent hydrogen or the group $—A—C_mH_{2m}—R^7$ or $—A—C_mH_{2m-2}—R^7$,

$R^7$ is hydrogen, unsubstituted phenyl or phenyl substituted by a $C_{1-3}$-alkyl, $C_{1-3}$-alkoxy or trifluoromethyl group, or a halogen atom,

A is a bond, —CO—, —COO— or $—CONR^8$,

$R^8$ is hydrogen or $C_{1-4}$-alkyl,

m is a numeral from 0 to 20 and

n is a numeral from 2 to 4,

characterized in that a 2.3-epoxypropylether of formula

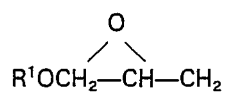

wherein $R^1$ is as defined in formula I, is reacted with an amine of formula

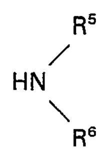

wherein $R^5$ and $R^6$ are as defined in formula I, after optional N-acylation, reacting the resulting compound of the formula II

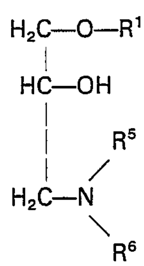

II

wherein $R^1$, $R^5$ and $R^6$ are as defined in formula I, with a dichlorophosphoric acid ω-haloalkyl ester of formula III

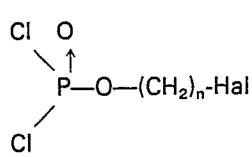

III

wherein n is as defined in formula I and Hal is a chlorine or bromine atom, in an inert organic solvent,

optionally in the presence of an auxiliary base, and reacting the resulting product with an amine of formula IV

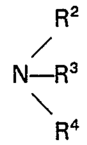

IV

wherein $R^2$, $R^3$ and $R^4$ are as defined in formula I, in an inert organic solvent, optionally under pressure, and, optionally if $R^5$ and $R^6$ in the resulting product of formula I is hydrogen, the product of formula I with $R^5$ and/or $R^6$ being hydrogen is reacted with an acid of formula V

$$HOOC—(CH_2)_m—R^7 \qquad V$$

wherein m and $R^7$ are as defined in formula I, in the presence of suitable condensation agents or, optionally in the presence of auxiliary bases, with an acid derivative of formula VI

$$Hal\text{-}A—(CH_2)_m—R^7 \qquad VI$$

wherein A, m and $R^7$ are as defined in formula I, except A does not represent a bond, and Hal is a halogen atom or an acid anhydride group, or, optionally in the presence of a Lewis base as catalyst, is reacted with an isocyanate of formula VII

$$R^9—N{=}C{=}O \qquad VII$$

wherein $R^9$ is $R^7C_mH_{2m}—$ or $R^7C_mH_{2m-2}—$, wherein $R^7$ and m are as defined in formula I.

**Patentansprüche für die Vertragstaaten: BE CH DE FR GB IT LI LU NL SE**

1. 1-O-Alkyl-3-aminopropan-1,2-diol-2-O-phospholipide der allgemeinen Formel I

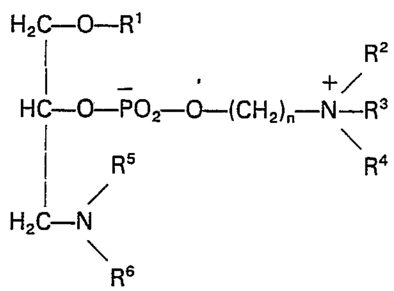

I

worin

$R^1$ einen gesättigten oder ungesättigten, geradkettigen oder verzweigten acyclischen Kohlenwasserstoffrest mit 10 bis 20 Kohlenstoffatomen ist,

$R^2$, $R^3$ und $R^4$, die gleich oder voneinander verschieden sein sein können, Wasserstoff oder einen Alkylrest mit 1 bis 4 Kohlenstoffatomen sind,

$R^5$ und $R^6$, die gleich oder voneinander verschieden sein können, Wasserstoff oder der Rest $—A—C_mH_{2m}—R^7$ oder $—A—C_mH_{2m-2}—R^7$ sind,

$R^7$ Wasserstoff, unsubstituiertes Phenyl oder durch ein $C_{1-3}$-Alkyl, $C_{1-3}$-Alkoxy oder Trifluormethyl oder Halogen substituiertes Phenyl ist,

A eine Bindung, —CO—, —COO— oder $—CONR^8—$ ist,

$R^8$ Wasserstoff oder $C_{1-4}$-Alkyl ist,

m eine ganze Zahl von 0 bis 20 ist und

n eine ganze Zahl von 2 bis 4 bedeutet.

2. 1-O-Alkyl-3-aminopropan-1,2-diol-2-O-phospholipide gemäß Anspruch 1, dadurch gekennzeichnet, daß in der allgemeinen Formel I

$R^1$ ein gesättigter oder ungesättigter, geradkettiger acyclischer Kohlenwasserstoffrest mit 10 bis 20 Kohlenstoffatomen ist,

$R^2$, $R^3$ und $R^4$, die gleich oder voneinander verschieden sein sein können, Wasserstoff oder Methyl sind,

$R^5$ und $R^6$, die gleich oder voneinander verschieden sein können, Wasserstoff oder der Rest $—A—(CH_{2m}—R^7$ sind,

$R^7$ Wasserstoff, unsubstituiertes Phenyl oder durch ein Methyl, Methoxy, Halogen oder Trifluormethyl substituiertes Phenyl ist,

A eine Bindung, —CO—, —COO— oder —$CONR^8$— ist,

$R^8$ Wasserstoff oder $C_{1-4}$-Alkyl ist,

m eine ganze Zahl von 0 bis 20 ist, wenn $R^7$ Wasserstoff ist, oder eine ganze Zahl von 0 bis 2 ist, wenn $R^7$ unsubstituiertes oder substituiertes Phenyl ist, und

n 2 bedeutet.

3. Verfahren zur Herstellung von Verbindungen der Formel I gemäß Anspruch 1 oder 2, dadurch gekennzeichnet, daß man einen 2,3-Epoxypropylether der Formel

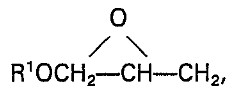

in der $R^1$ die gleiche Bedeutung wie in Anspruch 1 oder 2 hat, mit einem Amin der Formel

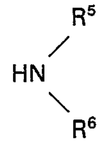

in der $R^5$ und $R^6$ die gleiche Bedeutung wie in Anspruch 1 oder 2 haben, und gegebenenfalls nachfolgender N-Acylierung zu einer Ausgangsverbindung der allgemeinen Formel II

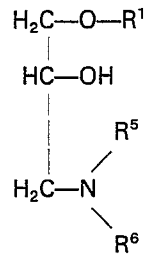

II

worin $R^1$, $R^5$ und $R^6$ die gleiche Bedeutung wie in Anspruch 1 oder 2 besitzen, umsetzt und diese mit einem Dichlorphosphorsäure-ω-haloalkylester der Formel III

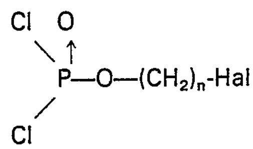

III

worin n die gleiche Bedeutung wie in Anspruch 1 oder 2 hat und Hal ein Chlor- oder Bromatom ist, in einem indifferenten organischen Lösungsmittel, ggfs. unter Anwendung einer Hilfsbase, umsetzt und die erhaltene Verbindung sodann mit einem Amin der Formel IV

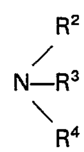

IV

worin $R^2$, $R^3$ und $R^4$ die gleiche Bedeutung wie in Anspruch 1 oder 2 besitzen, in einem indifferenten organischen Lösungsmittel, gegebenenfalls unter Druck, umsetzt.

4. Verfahren zur Herstellung von Verbindungen der Formel I gemäß Anspruch 1 oder 2, in denen $R^5$ und/oder $R^6$ Wasserstoff sind, dadurch gekennzeichnet, daß man eine Verbindung der Formel I, in der $R^5$ und/oder $R^6$ eine Benzyl- oder Benzyloxycarbonylgruppe sind, in einem indifferenten organischen Lösungsmittel in Gegenwart eines üblichen Hydrierkatalysators hydriert.

5. Verfahren zur Herstellung von Verbindungen der Formel I gemäß Anspruch 1 oder 2, dadurch gekennzeichnet, daß man eine Verbindung der Formel I, in der $R^5$ und/oder $R^6$ Wasserstoff sind, in Gegenwart eines üblichen geeigneten Kondensationsmittels mit einer Säure der Formel V

$$HOOC{-}(CH_2)_m{-}R^7 \qquad V$$

worin m und $R^7$ die gleiche Bedeutung wie in Anspruch 1 oder 2 haben, oder, gegebenenfalls in Gegenwart einer Hilfsbase, mit einem Säurederivat der Formel VI

$$Hal\text{-}A{-}(CH_2)_m{-}R^7 \qquad VI$$

worin A —CO—, —COO— oder —CONR$^8$— ist und m, $R^7$ und $R^8$ die gleiche Bedeutung wie in Anspruch 1 oder 2 haben und Hal ein Halogenatom ist, oder mit dem entsprechenden Säureanhydrid oder, gegebenenfalls unter Zusatz einer Lewis-Base als Katalysator, mit einem Isocyanat der Formel VII

$$R^9{-}N{=}C{=}O \qquad VII$$

worin $R^9 = R^7C_mH_{2m}$— oder $R^7C_mH_{2m-2}$— ist und $R^7$ und m die gleiche Bedeutung wie in Anspruch 1 haben, umsetzt.

6. Pharmazeutische Präparate, dadurch gekennzeichnet, daß sie eine Verbindung der allgemeinen Formel I gemäß Anspruch 1 oder 2 als Wirkstoff im Gemisch mit üblichen pharmazeutischen Hilfs- und Trägerstoffen enthalten.

**Patentanspruch für den Vertragsstaat: AT**

Verfahren zur Herstellung von Verbindungen der Formel I

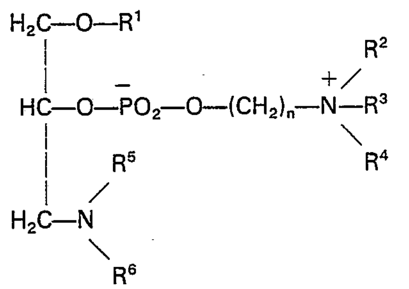

I

worin

$R^1$ einen gesättigten oder ungesättigten, geradkettigen oder verzweigten acyclischen Kohlenwasserstoffrest mit 10 bis 20 Kohlenstoffatomen ist,

$R^2$, $R^3$ und $R^4$, die gleich oder voneinander verschieden sein sein können, Wasserstoff oder einen Alkylrest mit 1 bis 4 Kohlenstoffatomen sind,

$R^5$ und $R^6$, die gleich oder voneinander verschieden sein können, Wasserstoff oder der Rest —A—$C_mH_{2m}$—$R^7$ oder —A—$C_mH_{2m-2}$—$R^7$ sind,

$R^7$ Wasserstoff, unsubstituiertes Phenyl oder durch ein $C_{1-3}$-Alkyl, $C_{1-3}$-Alkoxy oder Trifluormethyl oder Halogen substituiertes Phenyl ist,

A eine Bindung, —CO—, —COO— oder —CONR$^8$— ist,

$R^8$ Wasserstoff oder $C_{1-4}$-Alkyl ist,

m eine ganze Zahl von 0 bis 20 ist und

n eine ganze Zahl von 2 bis 4 bedeutet,

dadurch gekennzeichnet, daß man einen 2,3-Epoxypropylether der Formel

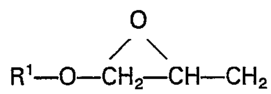

in der $R^1$ die gleiche Bedeutung wie Formel I hat, mit einem Amin der Formel

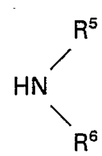

in der $R^5$ und $R^6$ die gleiche Bedeutung wie Formel I haben, umsetzt und gegebenenfalls nachfolgend N-acyliert zu einem Reaktionsprodukt der allgemeinen Formel II

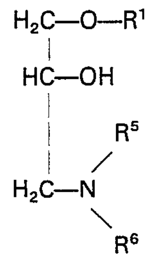

II

worin $R^1$, $R^5$ und $R^6$ die gleiche Bedeutung wie in Formel I haben, und diese mit einem Dichlorphosphorsäure-ω-haloalkylester der Formel III

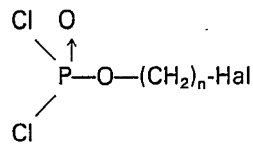

III

worin n die gleiche Bedeutung wie in Formel I hat und Hal ein Chlor- oder Bromatom ist, in einem indifferenten organischen Lösungsmittel, ggfs. unter Anwendung einer Hilfsbase, umsetzt und die erhaltene Verbindung sodann mit einem Amin der Formel IV

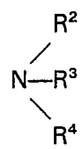

IV

worin $R^2$, $R^3$ und $R^4$ die gleiche Bedeutung wie in Formel I haben, in einem indifferenten organischen Lösungsmittel, gegebenenfalls unter Druck, umsetzt, und gegebenenfalls, wenn $R^5$ und $R^6$ der Formel I Wasserstoff sind, in Gegenwart eines üblichen geeigneten Kondensationsmittels mit einer Säure der Formel V

$$HOOC—(CH_2)_m—R^7 \qquad V$$

worin m und $R^7$ die gleiche Bedeutung wie in Formel I haben, oder, gegebenenfalls in Gegenwart einer Hilfsbase, mit einem Säurederivat der Formel VI

$$Hal\text{-}A—(CH_2)_m—R^7 \qquad VI$$

worin A, m, und $R^7$ die gleiche Bedeutung wie in Formel I haben und Hal ein Halogenatom ist, oder mit dem entsprechenden Säureanhydrid oder, gegebenenfalls unter Zusatz einer Lewis-Base als Katalysator, mit einem Isocyanat der Formel VII

$$R^9—N{=}C{=}O \qquad VII$$

worin $R^9 = R^7C_mH_{2m}—$ oder $R^7C_mH_{2m-2}—$ ist und $R^7$ und m die gleiche Bedeutung wie in Formel I haben, umsetzt.

**Revendications pour les Etats contractants: BE CH DE FR GB IT LI LU NL SE**

1. 1-O-Alkyl-3-aminopropane-1,2-diol-2-O-phospholipides de formule I

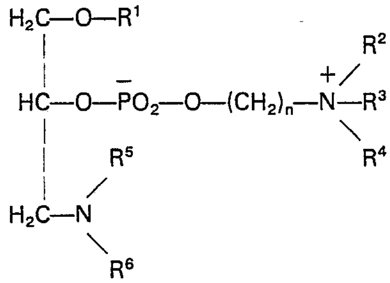

I

dans laquelle

$R^1$ est un groupe hydrocarboné acyclique à chaîne droite ou ramifiée, saturé ou insaturé, comportant 10 à 20 atomes de carbone

$R^2$, $R^3$ et $R^4$, qui peuvent être identiques ou différents, représentent un hydrogène ou un groupe alkyle ayant 1 à 4 atomes de carbone,

$R^5$ et $R^6$, qui peuvent être identiques ou différents, représentent un hydrogène ou le groupe $—A—C_mH_{2m}—R^7$ ou $—A—C_mH_{2m-2}—R^7$,

$R^7$ est un hydrogène, un groupe phényle non substitué ou phényle substitué par un groupe alkyle en $C_{1-3}$, alcoxy en $C_{1-3}$ ou trifluorométhyle, ou un atome d'halogène,

A est une liaison, —CO—, —COO— ou $—CONR^8—$,

$R^8$ est un hydrogène ou un groupe alkyle en $C_{1-4}$,

m est un nombre de 0 à 20 et

n est un nombre de 2 à 4.

2. 1-O-Alkyl-3-aminopropane-1,2-diol-2-O-phospholipides de formule générale I selon la revendication 1, dans laquelle

$R^1$ est un groupe hydrocarboné acyclique à chaîne droite, saturé ou insaturé, de 10 à 20 atomes de carbone,

$R^2$, $R^3$ et $R^4$, qui peuvent être identiques ou différents, représentent un hydrogène ou un groupe méthyle,

$R^5$ et $R^6$, qui peuvent être identiques ou différents, représentent un hydrogène ou le groupe $—A—(CH_2)_m—R^7$,

$R^7$ est un hydrogène, un groupe phényle non substitué ou phényle substitué par un groupe méthyle, méthoxy ou trifluorométhyle ou un atome d'halogène,

A est un liaison, —CO—, —COO—, ou $—CONR^8—$,

$R^8$ est un hydrogène ou un groupe alkyle en $C_{1-4}$,

m est un nombre de 0 à 20, si $R^7$ est un hydrogène, ou un nombre de 0 à 2, si $R^7$ est un groupe phényle ou phényle substitué et

n est égal à 2.

3. Procédé de production des composés de formule I selon les revendications 1 et 2, caractérisé en ce que l'on fait réagir un éther 2,3-époxypropylique de formule

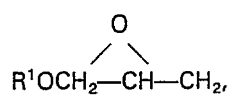

dans laquelle $R^1$ est tel que défini dans les revendications 1 et 2, avec une amine de formule

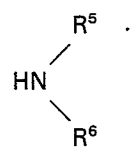

dans laquelle $R^5$ et $R^6$ sont tels que définis dans les revendications 1 et 2, après une N-acylation éventuelle, on fait réagir le composé résultant de formule II

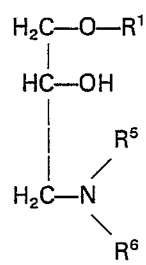

II

dans laquelle $R^1$, $R^5$ et $R^6$ sont tels que définis dans les revendications 1 et 2, avec un ester ω-halogéno-alkylique d'acide dichlorophosphorique de formule III

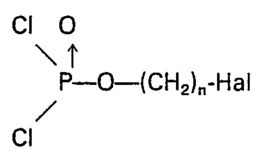

III

dans laquelle n est tel que défini dans les revendications 1 et 2 et Hal est un atome de chlore ou de brome,

dans un solvant organique inerte, éventuellement en présence d'une base auxiliaire, et on fait réagir le produit résultant avec une amine de formule IV

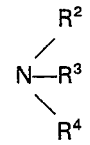

IV

dans laquelle $R^2$, $R^3$ et $R^4$ sont tels que définis dans les revendications 1 et 2, dans un solvant organique inerte, éventuellement sous pression.

4. Procédé de préparation des composés de formule I selon la revendication 1 ou 2, dans laquelle $R^5$ et/ou $R^6$ sont un hydrogène, caractérisé en ce que l'on soumet un composé de formule I dans laquelle un, ou les deux des groupes $R^5$ et/ou $R^6$ représentent un groupe benzyle ou benzyloxycarbonyle, dans un solvant inerte, en présence d'un catalyseur d'hydrogénation.

5. Procédé de production de composés de formule I selon la revendication 1 ou 2, caractérisé en ce que l'on fait réagir un composé de formule I dans laquelle $R^5$ et/ou $R^6$ sont un hydrogène, avec un acide de formule V

$$HOOC—(CH_2)_m—R^7 \qquad V$$

dans laquelle m et $R^7$ sont tels que définis dans les revendications 1 et 2, en présence d'agents de condensation convenables ou, éventuellement, en présence de bases auxiliaires avec un dérivé d'acide de formule VI

$$Hal\text{-}A—(CH_2)_m—R^7 \qquad VI$$

dans laquelle A, m et $R^7$ sont tels que définis dans les revendications 1 et 2, sauf que A ne représente pas une liaison, et Hal est un atome d'halogène ou un groupe anhydride d'acide ou, éventuellement, en présence d'une base de Lewis comme catalyseur, on le fait réagir avec un isocyanate de formule VII

$$R^9—N{=}C{=}O \qquad VII$$

dans laquelle $R^9$ est $R^7C_mH_{2m}$— ou $R^7C_mH_{2m-2}$—, où $R^7$ et m sont tels que définis dans la revendication 1.

6. Préparations pharmaceutiques, caractérisées en ce qu'elles contiennent un composé de formule générale I selon les revendications 1 et 2 comme substance active, mélangé avec des auxiliaires et des véhicules pharmaceutiques courants.

**Revendication pour l'Etat contractant: AT**

Procédé de production des composés de formule I

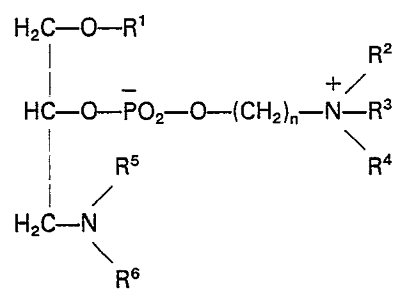

I

dans laquelle

$R^1$ est un groupe hydrocarboné acyclique à chaîne droite ou ramifiée, saturé ou insaturé, comportant 10 à 20 atomes de carbone

$R^2$, $R^3$ et $R^4$, qui peuvent être identiques ou différents, représentent un hydrogène ou un groupe alkyle ayant 1 à 4 atomes de carbone,

$R^5$ et $R^6$, qui peuvent être identiques ou différents, représentent un hydrogène ou le groupe —A—$C_mH_{2m}$—$R^7$ ou —A—$C_mH_{2m-2}R^7$,

$R^7$ est un hydrogène, un groupe phényle non substitué ou phényle substitué par un groupe alkyle en $C_{1-3}$, alcoxy en $C_{1-3}$ ou trifluorométhyle, ou un atome d'halogène,

A est une liaison, —CO—, —COO— ou —CONR$^8$—,

$R^8$ est un hydrogène ou un groupe alkyle en $C_{1-4}$,

m est un nombre de 0 à 20 et
n est un nombre de 2 à 4,

caractérisé en ce que l'on fait réagir un éther 2,3-époxypropylique de formule

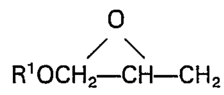

dans laquelle $R^1$ est tel que défini dans la formule I, avec une amine de formule

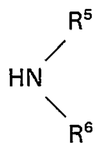

dans laquelle $R^5$ et $R^6$ sont tels que définis dans la formule I, après une N-acylation éventuelle, on fait réagir le composé résultant de formule II

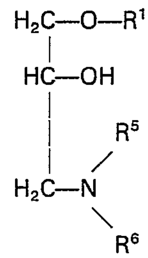

II

dans laquelle $R^1$, $R^5$ et $R^6$ sont tels que définis dans la formule I, avec un ester ω-halogénoalkylique d'acide dichlorophosphorique de formule III

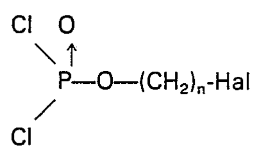

III

dans laquelle n est tel que défini dans la formule I et Hal est un atome de chlore ou de brome, dans un solvant organique inerte, éventuellement en présence d'une base auxiliaire, et on fait réagir le produit résultant avec une amine de formule IV

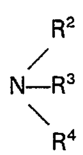

IV

dans laquelle $R^2$, $R^3$ et $R^4$ sont tels que définis dans la formule I, dans un solvant organique inerte, éventuellement sous pression, et, éventuellement, si $R^5$ et $R^6$, dans le produit résultant de formule I, sont un atome d'hydrogène, on fait réagir le produit de formule I, dans laquelle $R^5$ et/ou $R^6$ sont un hdyrogène, avec un acide de formule V

$$HOOC—(CH_2)_m—R^7 \qquad V$$

dans laquelle m et $R^7$ sont tels que définis dans la formule I, en présence d'agents de condensation convenables ou, éventuellement, en présence de bases auxiliaires, avec un dérivé d'acide de formule VI

$$Hal\text{-}A—(CH_2)_m—R^7 \qquad VI$$

dans laquelle A, m et $R^7$ sont tels que définis dans la formule I, sauf que A ne représente pas une liaison, et Hal est un atome d'halogène ou un groupe anhydride d'acide; ou, éventuellement, en présence d'une base de Lewis comme catalyseur, on le fait réagir avec un isocyanate de formule VII

$$R^9—N{=}C{=}O \qquad VII$$

dans laquelle $R^9$ est $R^7C_mH_{2m}—$ ou $R^7C_mH_{2m-2}—$, dans laquelle $R^7$ est m sont tels que définis dans la formule I.